# EUROPÄISCHE PATENTSCHRIFT

(11) **EP 0 879 018 B1**
(45) Veröffentlichungstag und Bekanntmachung des Hinweises auf die Patenterteilung: **23.05.2001**
(21) Anmeldenummer: 97908133.8
(22) Anmeldetag: 10.02.1997
(51) Int. Cl.: A61B 17/02

(54) **VORRICHTUNG ZUM ANHEBEN DER BAUCHDECKE FÜR DIE DURCHFÜHRUNG LAPAROSKOPISCHER UNTERSUCHUNGEN**
DEVICE FOR LIFTING THE ABDOMINAL WALL TO CARRY OUT LAPAROSCOPIC EXAMINATIONS
DISPOSITIF SERVANT A SOULEVER LA PAROI ABDOMINALE POUR LA REALISATION D'EXAMENS LAPAROSCOPIQUES

(30) Priorität: 08.02.1996 DE 19604618
(43) Veröffentlichungstag der Anmeldung: 25.11.1998
(73) Patentinhaber: Karl Storz GmbH & Co. KG, 78532 Tuttlingen (DE)
(72) Erfinder: KECKSTEIN, Jörg, A-9500 Villach (AT); BACHER, Uwe, D-78532 Tuttlingen (DE); LEHMANN, Markus, D-85356 Freising (DE)
(74) Vertreter: Münich, Wilhelm, Dr.
(86) Internationale Anmeldenummer: DE9700254
(87) Internationale Veröffentlichungsnummer: WO9728747

(56) Entgegenhaltungen:
- EP-A- 0 614 646
- DE-C- 640 126
- DE-U- 9 106 553
- US-A- 5 289 817

## Beschreibung

### Technisches Gebiet

Die Erfindung bezieht sich auf eine Vorrichtung zum Anheben der Bauchdecke für die Durchführung laparoskopischer Untersuchungen, die durch eine Öffnung in der Bauchdecke in den Bauchraum zumindest teilweise einführbar ist, gemäß dem Oberbegriff des Patentanspruchs 1.

### Stand der Technik

Gattungsgemäße Vorrichtungen weisen einen Instrumentenschaft auf, der an seinem distalen Endbereich wenigstens einen, vorzugsweise zwei parallel zur Instrumentenschaftachse angeordnete Schenkel vorsieht. Die Schenkel sind seitlich zur Schaftachse ausklappbar.

Vorrichtungen der obenstehenden Gattung, die auf dem Gebiet der Endoskop-Chirurgie zur Durchführung laparoskopischer Untersuchungen bzw. Behandlungen verwendet werden, sind auch unter dem Begriff "Elevationsinstrumente" bekannt. Derartige Instrumente werden durch eine enge Öffnung in den Bauchraum eingeführt und ermöglichen durch seitliches Ausklappen von Halteträgern eine weitgefächerte Auflage, um möglichst über einen großen Bereich die Bauchdecke anzuheben. Auf diese Weise kann der für nachfolgende laparoskopische Untersuchungen sowie endoskopisch unterstützte chirurgische Eingriffe benötigte Operationsraum innerhalb des Bauchlumens geschafft werden.

Die für die Aufweitung des Bauchraumes verwendeten Elevationsinstrumente sollen zum einen einen möglichst kleinen Querschnitt aufweisen, so daß die Öffnung in der Bauchdekke, durch die das Instrument in das Bauchinnere eingeführt wird, möglichst klein gehalten werden kann, zum anderen sollen jedoch die seitlich von dem Instrument ausklappbaren Tragarme oder im folgenden Schenkel genannt, eine möglichst weitgefächerte Auflage für die Bauchdecke bieten.

An den Schwenk- bzw. Klappmechanismus, der die Schenkel von der Instrumentenachse wegklappt, sind folgende Anforderungen zu stellen:

Der Aus- und Einklappvorgang der Schenkel soll zuverlässig und dosiert innerhalb des Bauchraumes erfolgen. Der gesamte Tragemechanismus soll über eine ausreichende Stabilität zum Tragen der Bauchdeckenlast aufweisen und zugleich einen geringen Platzbedarf beanspruchen. Ferner sollen Sicherheitseinrichtungen vorgesehen sein, um das Elevationsinstrument bei Versagen des Klappmechanismuses wieder aus dem Bauchraum entfernen zu können.

In dem Artikel von Albert K. Chin et al. "Gasless Laparoscopy using a planer lifting technic", erschienen in Journal of the American College of Surgion, April 1994, Vol. 178, Seiten 401-403 ist eine gattungsgemäße Vorrichtung zur mechanischen Anhebung der Bauchdecke beschrieben, mit der es möglich ist, laparoskopische Operationen ohne andauernde Insufflation von Gas in den Bauchraum durchzuführen. Die durch die Insufflation von vorzugsweise Kohlendioxid entstehende Gasblase innerhalb des Bauchraumes kann zum einen durch die künstliche Aufrechterhaltung eines relativ hohen Druckes zu einer Verminderung der Atmungskapazität sowie zu einem verminderten venösen Rückfluß führen. Um diese und weitere Nachteile für den Patienten zu vermeiden, werden die gattungsgemäßen Elevationsinstrumente eingesetzt.

Die in dem vorstehend genannten Artikel beschriebene gattungsgemäße Vorrichtung sieht einen Handhabungsschaft vor, an dessen distalseitigem Ende im rechten Winkel zur Schaftachse eine Spreizvorrichtung angebracht ist, die zur Einführung in den Bauchraum eine geschlossene Stellung einnimmt, so daß die Spreizvorrichtung durch sehr enge Bauchöffnungen in den Bauchraum verbracht werden kann. Durch eine am Handhabungsschaft angebrachte Betätigungsmechanik kann der Klappmechanismus innerhalb des Bauchraumes scherenförmig aufgespreizt werden, wodurch zwei in V-Stellung vorgesehene Schenkel durch entsprechendes Anheben des Instrumentes die Bauchdecke intrakorporal nach oben heben.

Eine Wiederentnahme des Gerätes ist erst möglich, nachdem die in V-Stellung eingestellten Schenkel durch den entsprechenden Bedienmechanismus am Handhabungsschaft in eine Parallelstellung zurückgeführt werden. Sind jedoch zwischen den Schenkeln Gewebeteile vorhanden, so können diese bei der Wiederzusammenführung der Schenkel entsprechend gequetscht oder irreversibel beschädigt werden. Überdies ist in einem solchen Falle eine vollständige Zusammenführung der beiden Schenkel nicht möglich, wodurch die schonende Wiederentnahme des Instrumentes durch die kleine Bauchöffnung erheblich beeinträchtigt ist.

Ferner sind gattungsgemäße Vorrichtungen bekannt, die parallel zur Instrumentenschaftachse ausklappbare Schenkel vorsehen, die nach Einbringung in den Bauchraum gleichsam dem Mechanismus eines Regenschirmes innerhalb des Bauchraumes ausklappen und bei entsprechender Anhebung des Instrumentes die Bauchdecke intrakorporal von unten in Richtung der Instrumentenbewegung anheben. Auch bei derartigen Klappmechanismen, die vorzugsweise einen Schwenkbereich zwischen der Parallelstellung zur Schaftachse und eine Orthogonalstellung zur Schaftachse einnehmen, besteht das Problem, daß das Wiedereinklappen der Schenkel durch im Bauchraum vorhandene Gewebeteile beeinträchtigt werden kann. Ein nicht vollständiges Wiedereinklappen der einzelnen Schenkel führt jedoch zu einer Art Wiederhaken, dessen Entnahme durch die kleine Bauchöffnung mit Beschädigungen des Gewebes, das die Bauchöffnung unmittelbar umgibt, unvermeidbar verbunden ist.

Eine Vorrichtung mit den Merkmalen des Oberbegriffs des Anspruchs 1 ist aus DE-C-640 126 bekannt.

### Darstellung der Erfindung

Der Erfindung liegt die Aufgabe zugrunde, eine Vorrichtung zum Anheben der Bauchdecke für die Durchführung laparoskopischer Untersuchungen gemäß dem Oberbegriff des Anspruchs 1 derart weiterzubilden, daß die vorstehend beschriebene Gefahr, eines nur unvollständigen Wiedereinklappens der zum Anheben der Bauchdecke gespreizten Instrumentenschenkel, durch beispielsweise Verklemmen oder Einquetschen mit Gewebeteilen, weitgehend ausgeschlossen werden sollte. Hierfür sollte ein Klappmechanismus für das Ausklappen von Spreizschenkeln verwendet werden, der möglichst wenig Platz beansprucht, um intrakorporale Gewebeirritationen zu vermeiden. Gleichwohl soll das Instrument leichtgängig und ohne großen Kraftaufwand zu bedienen sein.

Eine Lösung der Aufgabe ist im Anspruch 1 angegeben. Den Lösungsgedanken vorteilhaft ausbildende weitere Merkmale sind Gegenstand der Unteransprüche 2 ff.

Erfindungsgemäß ist eine Vorrichtung zum Anheben der Bauchdecke für die Durchführung laparoskopischer Untersuchungen gemäß dem Oberbegriff des Anspruchs 1 derart aus-gebildet, daß sie die Merkmale des Kennzeichnenden Teils des Anspruchs 1 aufweist.

Auf diese Weise ist es möglich, die zum Anheben in vorzugsweise Orthogonalstellung zum Instrumentenschaft ausgeklappten Schenkel für die Wiederentnahme des Instruments aus dem Bauchraum entweder zur Proximalseite des Instrumentes parallel zur Instrumentenschaftachse zu klappen oder, falls ein vollständiges Anliegen der Schenkel an die Instrumentenachse durch eingeklemmte Gewebeteile verhindert wird, entgegengesetzt dazu, d.h. distalseitig umzuklappen, bis die Schenkel parallel zur Instrumentenschaftachse anliegen, um das Instrument in dieser Stellung leicht durch die Bauchöffnung aus dem Bauchraum zu entnehmen.

### Kurze Beschreibung der Zeichnung

Die Beschreibung des erfindungsgemäßen 180°-Klappmechanismus soll anhand eines Ausführungsbeispiels unter Bezugnahme auf die Zeichnungen exemplarisch dargestellt werden. Es zeigen:
- Fig. 1: Prinzipskizze für den Einsatz eines erfindungsgemäßen Elevationsinstrumentes im Bauchraum eines Patienten,
- Fig. 2: Prinzipskizze eines erfindungsgemäßen Klappmechanismus,
- Fig. 3: schematisierte Darstellung des distalen Endes des Instrumentenschaftes
- Fig. 4: Draufsicht auf einen Schenkel mit eingearbeiteter Führungsbahn sowie
- Fig. 5: Darstellung der Kräfteverhältnisse in der Steuerkurve.

### Darstellung eines Ausführungsbeispiels

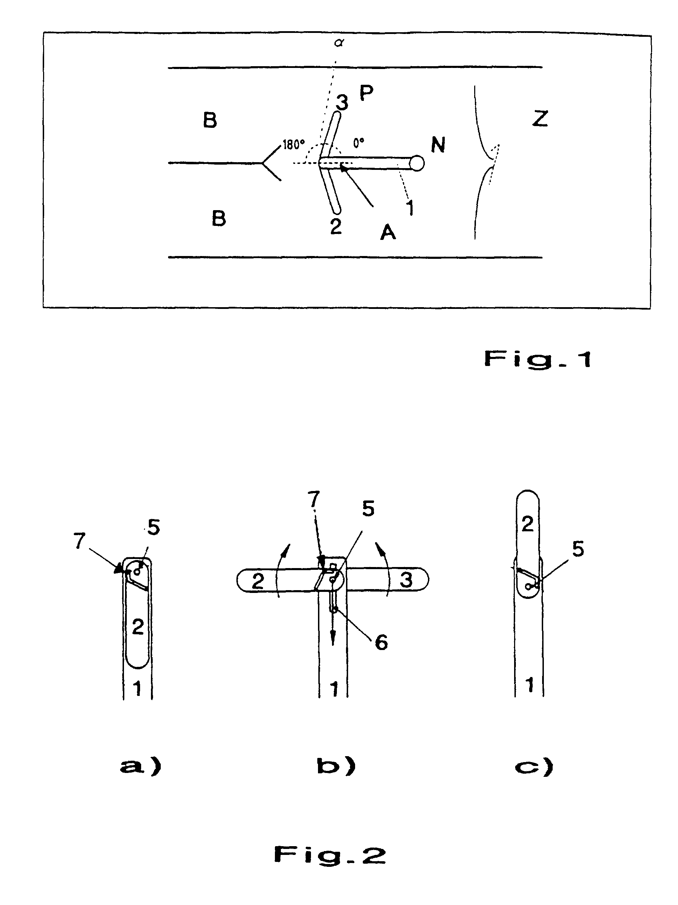

Aus Fig. 1 ist die schematisierte Draufsicht auf einen Patienten P, dessen Beine B sowie Zwerchfell Z eingezeichnet sind. Durch eine Körperöffnung N ist das Elevationsinstrument in das Körperinnere eingeführt. Das Elevationsinstrument weist einen Instrumentenschaft 1 sowie zwei seitlich zur Instrumentenschaftachse A ausklappbare Schenkel 2 und 3 auf. Erfindungsgemäß können die Schenkel 2 und 3 in einem Winkelbereich von 0° ≤ α ≤ 180° geschwenkt werden.

Die Stellung α = 0° entspricht dabei der Schenkelstellung zum Einführen des Instrumentes durch die Bauchöffnung N. Sollten die Schenkel aus irgendeinem Grunde innerhalb des Bauchraumes nicht mehr in die Ausgangsstellung zurückklappbar sein, sei es durch eingeklemmtes Gewebe oder mechanische Defekte, können beide Schenkel in die 180°-Stellung geklappt werden. Die Möglichkeit, das Instrument durch die Bauchraumöffnung N wieder zu entfernen, ist auf diese Weise in jedem Fall gegeben.

In den Teilbildern der Fig. 2a bis 2c ist das Grundprinzip des Klappmechanismus ersichtlich. Jeweils in der Seitendarstellung des distalen Endbereiches der erfindungsgemäßen Vorrichtung ist am Instrumentenschaft 1 in der Darstellung gemäß Fig. 2a ein Schenkel 2 in Parallelstellung zur Schaftachse dargestellt. In dieser Anordnung wird der Instrumentenschaft durch eine nicht in der Figur dargestellte Bauchöffnung in das Bauchinnere verbracht. Grundsätzlich weisen die ausklappbaren Schenkel 2 und 3 jeweils eine Steuerkurve 4 auf, die in jeweils einen Seitenendbereich eines jeden Schenkels eingearbeitet ist. Die Steuerkurve 4 kann als Nut oder als vollständig durch das Material des Schenkels hindurchragender Schlitz ausgestaltet sein.

Neben der Steuerkurve 4 weist jeder Schenkel eine drehbewegliche Verbindungseinrichtung 5 auf. Die an den Schenkeln angebrachte Verbindungseinrichtung 5 durchragt eine als Schlitz ausgebildete Führungsbahn 6, die im distalen Bereich des Instrumentenschaftes 1 eingearbeitet ist und verbindet auf diese Weise beide Schenkel 2 und 3 miteinander. Zusätzlich zur Führungsbahn 6 weist der distale Bereich des Instrumentenschaftes 1 pro Schenkelseite einen fest auf dem Instrumentenschaft angeordneten Steuerstift 7 auf, der die Steuerkurve 4 des jeweiligen Schenkels durchragt.

Mit Hilfe des sich daraus ergebenden Klappmechanismus können die Schenkel aus der Parallelstellung gemäß Fig. 2a durch proximales Verschieben der Verbindungseinrichtung entlang der Führungsbahn 6 in die Orthogonalstellung gemäß Fig. 2b überführt werden.

Durch die Axialverschiebung der Verbindungseinrichtung 5 entlang der Führungsbahn 6 erfährt jeder Schenkel aufgrund des Ineinandergreifens des Steuerstiftes 7 in die Steuerkurve ein Drehmoment, das die Schenkel um die Verbindungseinrichtung 5, die zugleich auch das Drehzentrum der Schenkel darstellt, eine durch die gebogenen Pfeile dargestellte Verkippung. Befindet sich die Verbindungseinrichtung 5 am proximalseitig zugewandten Ende der Führungsbahn 6, so sind beide Schenkel distalseitig parallel zur Instrumentenachse gerichtet (siehe hierzu Fig. 2c).

Die kinematische Ansteuerung der Verbindungseinrichtung 5 zur axialen Verschiebung erfolgt über ein nicht in den Figuren 2 dargestelltes Bedienelement, das in Form einer Schubstange ausgeführt sein kann. Je nach Stellung der Schenkel könne diese über die Schubstsnge in ihrer Lage arretiert werden.

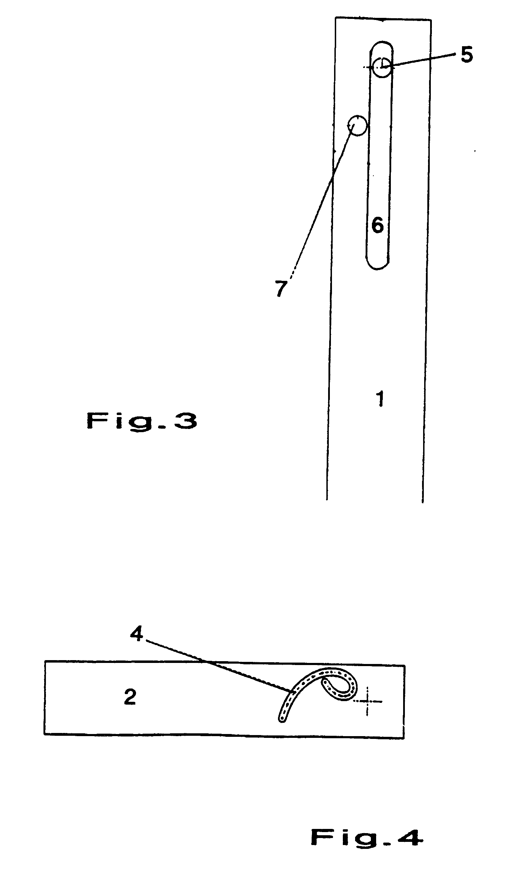

In Fig. 3 ist schematisiert eine vorteilhafte Ausführungsform des distalen Endbereiches eines Instrumentenschaftes 1 dargestellt. Seitlich neben der Führungsbahn 6 ist der Steuerstift fest angeordnet, der in die Steuerkurve des jeweiligen Schenkels eingreift. Die längsbewegliche Verbindungseinrichtung 5, die durch die Führungsbahn 6 des Instrumentenschafts hindurchragt und beide Schenkel kinematisch miteinander verbindet, kann über ein nicht dargestelltes Bedienelement in jeder Position innerhalb der Führungsbahn arretiert werden.

In Fig. 4 ist ein vorteilhaft ausgebildeter Schenkel 2 dargestellt, der ein Kreuz aufweist, das den Durchstoßpunkt der Verbindungseinrichtung 5 angibt. Die besonders vorteilhaft ausgebildete Steuerkurve ist dabei derart ausgebildet, daß sie vollständig im Schenkelbereich eingearbeitet ist und sich selbst nicht schneidet.

Mit der in Fig. 4 dargestellten Steuerkurvenform ist es insbesondere möglich, daß ein gleichmäßiges Verschieben der Verbindungseinrichtung zu einer gleichförmigen Bewegung der Schenkel um ihren Drehpunkt führt.

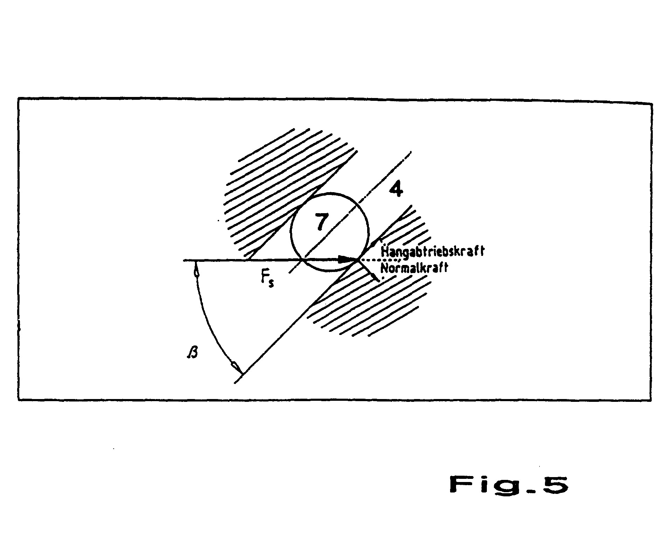

Die Klappbewegung der Schenkel wird durch ein Moment erzeugt, das durch die Führung des Steuerstiftes innerhalb der Steuerkurve entsteht. Durch die Gleitbewegung des Steuerstiftes innerhalb der Steuerkurve übt dieser eine Kraft FS auf die Außenkontur der Kurvennut aus. Dies ist in Fig. 5 dargestellt. Es entstehen die Kraftverhältnisse einer schiefen Ebene. Die Kraft FS teilt sich damit in die Komponenten Hangabtriebskraft FH und Normalkraft FN auf. Die Normalkraft FN ist dabei für die Bewegung der Schenkel verantwortlich. Je größer ihr Anteil im Kräfteplan ist, umso mehr Kraft wird für die Erzeugung des Momentes MS bereitgestellt. Mit steigender Normalkraft steigt aber auch die Flächenpressung zwischen Stift und Kontur der Steuerkurve und damit die bei der Bewegung aufzuwendende Reibungsarbeit.

Damit der Steuerstift die Kurvenkontur leicht durchläuft, darf also der Anteil der Komponente Hangabtriebskraft FH nicht zu klein gewählt werden. Der Winkel β sollte deshalb an keiner Stelle der Kurve größer als 65° sein. Auf diese Weise ist sichergestellt, daß mindestens 1/4 der aufgewendeten Kraft als Hangabtriebskraft auftritt.

## Patentansprüche

1. Vorrichtung zum Anheben der Bauchdecke für die Durchführung laparoskopischer Untersuchungen, die durch eine öffnung (N) in der Bauchdecke in den Bauchraum einführbar ist, mit
- einem Instrumentenschaft (1) mit einer longitudinalen Schaftachse (A),
- wenigstens einem, vorzugsweise zwei Schenkeln (2,3), die in einer Einführungsstellung parallel zur Achse des Instrumentenschaftes angeordnet sind, und um eine zur Schaftachse senkrechte Achse im distalen Endbereich des Instrumentenschafts schwenkbar sind,
- und einem Klappmechanismus, der den bzw. die Schenkel über einen Winkelbereich von bis zu 180° schwenkt,
dadurch gekennzeichnet, daß der Klappmechanismus eine im distalen Endbereich des Instrumentenschafts, axial zur Schaftachse verlaufende Führungsbahn (6) aufweist, und
daß jeder Schenkel in einem seiner Seitenendbereiche eine Steuerkurve (4) sowie eine stiftartige Verbindungseinrichtung aufweist, die den Schenkel drehbar um die Verbindungseinrichtung und entlang der Führungsbahn des Instrumentenschaftes bewegbar, mit dem Instrumentenschaft fest verbindet.

2. Vorrichtung nach Anspruch 1,
dadurch **gekennzeichent,** daß die Führungsbahn (6) eine Nut oder ein den Instrumentenschaft vollständig durchsetzender Führungsschlitz ist.

3. Vorrichtung nach Anspruch 1 oder 2,
dadurch **gekennzeichnet,** daß der Schenkel von abgeflachter Stabform ist und eine Breite aufweist, die maximal der distalen Instrumentenschaftbreite entspricht.

4. Vorrichtung nach einem der Ansprüche 1 bis 3,
dadurch **gekennzeichnet,** daß die Steuerkurve mit einem auf dem Instrumentenschaft fest angebrachten Steuerstift (7) in Eingriff steht.

5. Vorrichtung nach einem der Ansprüche 1 bis 4,
dadurch **gekennzeichnet,** daß die Steuerkurve eine gebogene Kontur aufweist, die sich selbst nicht schneidet.

6. Vorrichtung nach einem der Ansprüche 1 bis 5,
dadurch **gekennzeichnet,** daß die Steuerkurve als Schlitz ausgebildet ist.

7. Vorrichtung nach einem der Ansprüche 1 bis 6,
dadurch **gekennzeichnet,** daß der Klappmechanismus durch Verschieben der Verbindungseinrichtung (5) entlang der Führungsbahn (6) des Instrumentenschaftes eine seiltiche Auslenkung der Schenkel (2, 3) durch die erfolgende Relativbewegung des Steuerstiftes (7) innerhalb der Steuerkurve (4) bewirkt.

8. Vorrichtung nach einem der Ansprüche 1 bis 7,
dadurch **gekennzeichnet,** daß ein Bedienelement vorgesehen ist, das den Klappmechanismus von der Proximalseite des Instrumentenschaftes ansteuert.

9. Vorrichtung nach Anspruch 8,
dadurch **gekennzeichnet,** daß das Bedienelement mit der Verbindungseinrichtung in Wirkverbindung steht.

10. Vorrichtung nach einem der Ansprüche 1 bis 9,
dadurch **gekennzeichnet,** daß die Schenkel wenigstens in einer Stellung senkrecht zur Instrumentenschaftachse arretierbar sind.

11. Vorrichtung nach einem der Ansprüche 1 bis 10,
dadurch **gekennzeichnet,** daß zwei an einem Instrumentenschaft angeordnete Schenkel über eine stiftartige Verbindungseinrichtung miteinander drehbar fest verbunden sind, die zugleich eine gemeinsame Drehachse der Schenkel bildet.

## Claims

1. Device for lifting the abdominal wall to carry out laparoscopic examinations, which is adapted to be introduced, at least in part, through an opening (N) in the abdominal wall, comprising
- an instrument shaft (1) having a longitudinal shaft axis (A),
- at least one, preferable two branches (2, 3) disposed, in an introducing position, in parallel with the axis of said instrument shaft and pivotable about an axis orthogonal on said shaft axis in the distal terminal region of said instrument shaft,
- and a swinging mechanism which pivots said branch or branches, respectively, within an angular range of up to 180°,
**characterised** in that said swinging mechanism comprises a guideway (6) extending axially with respect to said shaft axis in the distal terminal region of said instrument shaft, and
that each branch comprises a cam (4) in one of its lateral terminal regions as well as a pin-type connecting means which establishes a fixed connection of said branch for rotation about said connecting means and for movement along said guideway of said instrument shaft.

2. Device according to Claim 1,
**characterised** in that said guideway (6) is a groove or a guiding slot passing completely through said instrument shaft.

3. Device according to Claim 1 or 2,
**characterised** in that said branch has the shape of a flattened rod and presents a width corresponding, at maximum, to the distal width of said instrument shaft.

4. Device according to any of the Claims 1 to 3,
**characterised** in that said cam is engaged in a control pin (7) fixedly attached on said instrument shaft.

5. Device according to any of the Claims 1 to 4,
**characterised** in that said cam has a curved contour that does not intersect with itself.

6. Device according to any of the Claims 1 to 5,
**characterised** in that said cam is configured as a slot.

7. Device according to any of the Claims 1 to 6,
**characterised** in that by displacement of said connecting means (5) along said guideway (6) of said instrument shaft, said swinging mechanism causes a lateral deflection of said branches (2, 3) due to the relative movement of said control pin (7) which then takes place within said cam (4).

8. Device according to any of the Claims 1 to 7,
**characterised** in that an operating element is provided for controlling said swinging mechanism from the proximal side of said instrument shaft.

9. Device according to Claim 8,
**characterised** in that said operating element is operatively connected to said connecting means.

10. Device according to any of the Claims 1 to 9,
**characterised** in that said branches are adapted to be arrested in at least one position orthogonal on the axis of said instrument shaft.

11. Device according to any of the Claims 1 to 10,
**characterised** in that two branches disposed on an instrument shaft are fixedly interconnected for rotation via a pin-like connecting means that constitutes, at the same time, a common axis of rotation of said branches.

## Revendications

1. Dispositif à soulever la paroi abdominale pour la réalisation d'examens laparoscopiques, qui est approprié à être introduit, au moins en partie, à travers une ouverture (N) dans la paroi abdominale, comprenant
- une tige d'instrument (1) à un axe de la tige (A) longitudinal,
- au moins une branche, de préférence deux branches (2, 3) disposées, en position d'introduction, en parallèle à l'axe de ladite tige d'instrument et pivotables autour d'un axe orthogonal sur ledit axe de la tige dans la zone terminale distale de ladite tige d'instrument,
- et un mécanisme de pivotement, qui pivote ladite branche ou respectivement lesdites branches au-dedans une gamme angulaire jusqu'à 180°,
**caractérisé** en ce que ledit mécanisme de pivotement comprend une glissière de guidage (6), qui s'étend en sens axial relativement audit axe de la tige dans la zone terminale distale de ladite tige d'instrument, et
en ce que chaque branche comprend une came de commande (4) dans une de ses régions terminales latérales, ainsi qu'un moyen de connexion du type d'une cheville, qui établit une connexion fixe de ladite branche pour rotation autour dudit moyen de connexion et pour mouvement le long de ladite glissière de guidage de ladite tige d'instrument.

2. Dispositif selon la revendication 1,
**caractérisé** en ce que ladite glissière de guidage (6) est une rainure ou une fente de guidage, qui s'étend complètement à travers de ladite tige d'instrument.

3. Dispositif selon la revendication 1 ou 2,
**caractérisé** en ce que ladite branche a la forme d'une barre aplatie et une largeur qui correspond, au maximum, à la largeur distale de ladite tige d'instrument.

4. Dispositif selon une quelconque des revendications 1 à 3,
**caractérisé** en ce que ladite came de commande s'engage dans une cheville de commande (7) fixée sur ladite tige d'instrument.

5. Dispositif selon une quelconque des revendications 1 à 4,
**caractérisé** en ce que ladite came de commande a un contour courbée, que ne coupe pas soi-même.

6. Dispositif selon une quelconque des revendications 1 à 5,
**caractérisé** en ce que ladite came de commande est configurée sous forme d'une fente.

7. Dispositif selon une quelconque des revendications 1 à 6,
**caractérisé** en ce que par déplacement dudit moyen de connexion (5) le long de ladite glissière de guidage (6) de ladite tige d'instrument, ledit mécanisme de pivotement cause une déflexion latérale desdites branches (2, 3) en vertu du mouvement relatif de ladite cheville de commande (7), qui a lieu à l'intérieur de ladite came de commande (4).

8. Dispositif selon une quelconque des revendications 1 à 7,
**caractérisé** en ce qu'un élément de manoeuvre est disposé pour la commande dudit mécanisme de pivotement à partir du côté proximal de ladite tige d'instrument.

9. Dispositif selon la revendication 8,
**caractérisé** en ce que ledit élément de manoeuvre est activement relié audit moyen de connexion.

10. Dispositif selon une quelconque des revendications 1 à 9,
**caractérisé** en ce que lesdites branches sont aptes à être arrêtées dans au moins une position orthogonale sur l'axe de ladite tige d'instrument.

11. Dispositif selon une quelconque des revendications 1 à 10,
**caractérisé** en ce que deux branches disposées sur une tige d'instrument sont fixées et reliées l'une à l'autre pour rotation via un moyen de connexion du type d'une cheville, qui constitue, en même temps, un axe de rotation commun desdites branches.
